# EUROPEAN PATENT APPLICATION

(11) **EP 2 062 909 A1**
(43) Date of publication of application: **27.05.2009**
(21) Application number: 07121207.0
(22) Date of filing: 21.11.2007
(51) Int. Cl.: C07K 1/107, C07K 7/06

(54) **Peptide production and purification process**

(71) Applicant: SOLVAY (Société Anonyme), 1050 Bruxelles (BE)
(72) Inventor: Callens, Roland, 1850, Grimbergen (BE); Blondeel, Georges, 9300, Aalst (BE); Delplanche, Thierry, 1435, Mont Saint Guibert (BE)
(74) Representative: Mross, Stefan P.M.

(57) **Abstract**

Processes for the synthesis and purification of a Gly-Gly-Val-Leu-Val-Gln-Pro-Gly octapeptide (SEQ ID NO 1).

## Description

### Technical Field

The present invention relates to a process for the manufacture of a certain octapeptide, and in particular to such a process comprising a purification step.

### Background Art

The fact that *zonula occludens* toxin ("zot"), an enterotoxin produced by *Vibrio cholera,* increases permeability by reversibly affecting the structure of tight junctions was described for the first time in PCT/WO 9637196 (UNIVERSITY OF MARYLAND (US)). The comparison of its sequence with the one of its eukaryotic human analogue (zonulin) revealed an 8-amino acid shared motif in their putative binding domain (GXXXVGXG), described by DI PIERRO, et al. Zonula Occludens Toxin Structure-Function Analysis. J. biol. chem.. 2001, vol.276, no.22, p.19160-19165.

The octapeptide (present in zonulin) of the following sequence: Gly-Gly-Val-Leu-Val-Gln-Pro-Gly (SEQ ID NO 1)
has been described as a peptide antagonist of zonulin binding to zonula occludens toxin receptor by Fasano in PCT/WO 0007609 (UNIVERSITY OF MARYLAND (US)) and by WANG, et al. Human Zonulin, a Potential Modulator of Intestinal Tight Junctions. Journal of Cell Science. 2000, vol.113, p.4435-4440. Fasano discloses its application in methods for treatment of gastrointestinal inflammation as well as of conditions associated with breakdown of the blood brain barrier. Fasano *et al.* also describe in US 7,026,294 B (UNIVERSITY OF MARYLAND (US)) its use in a method for delay of onset of diabetes.

This octapeptide seems to be very promising in the field of the treatment of various diseases that involve disordered intercellular communication including developmental and intestinal disorders leading to autoimmune disease (coeliac disease and type 1 diabetes), tissue inflammation, malignant transformation, and metastasis. None of the above references describe any process for the synthesis of this octapeptide. The present invention makes now available a process for its manufacture.

The Applicant describes here a process for the synthesis of the octapeptide Gly-Gly-Val-Leu-Val-Gln-Pro-Gly (SEQ ID NO 1), which allows for an efficient production of said octapeptide with a good yield and a high quality purity level while presenting advantages in terms of productivity and required manufacturing equipment.

### Disclosure of Invention

### Synthesis of Gly-Gly-Val-Leu-Val-Gln-Pro-Gly (SEQ ID NO 1)

The present invention concerns a process for the synthesis of a Gly-Gly-Val-Leu-Val-Gln-Pro-Gly octapeptide (SEQ ID NO 1) comprising at least one peptide coupling step carried out in solution.

The process according to the present invention allows using a convergent synthetic strategy, with a limited number of steps, and avoids successive protection/deprotection reactions.

It has been found, surprisingly, that the process according to the invention allows providing said octapeptide in an industrial scale without substantial formation of by-products or racemisation. Moreover, the by-products possibly formed during the process according to the present invention are readily separable from the final octapeptide by a specific purification process. Besides, the present invention allows the synthesis of peptides containing both L- and D-amino acid configurations.

In a first particular aspect, the process according to the present invention comprises coupling a peptide of formula :

Val-Gln-Pro-Gly-Y (SEQ ID NO 2) ;

wherein the C-terminal amino acid is protected by a carboxylic acid protecting group Y, preferably selected from alkyl esters, aryl esters, aralkyl esters and silyl groups ;
with a Leucine or a C-terminal Leucine peptide, preferably selected from formulae:

X-Gly-Gly-Val-Leu (SEQ ID NO 3), X-Gly-Val-Leu and X-Val-Leu ;

wherein the N-terminal amino acid is protected by an amino protecting group X preferably selected from allyloxycarbonyl groups,
tert-butyloxycarbonyl (BOC), benzyloxycarbonyl (Z),
9-fluorenylmethyloxycarbonyl (Fmoc), 4-nitrobenzenesulfonyl (Nosyl), 2-nitrobenzenesulfenyl (Nps) and optionally substituted derivatives thereof; and wherein said Leucine or C-terminal Leucine peptide is optionally activated by a carboxylic acid activating agent.

For the purpose of the present invention, the term "peptide" refers to a polymer in which the monomers are amino acids covalently attached together through amide bonds. Peptides are two or often more amino acids monomers long. In addition, all peptide sequences are represented by formulae whose left to right orientation is in the conventional direction of amino-terminus to carboxy-terminus.

For the purpose of the present invention, the term "amino acid" is intended to denote any compound comprising at least one NR1R2 group, preferably NH₂ group, and at least one carboxyl group. The amino acids of this invention can be naturally occurring or synthetic. The natural amino acids, with exception of glycine, contain a chiral carbon atom. Unless otherwise specifically indicated, the compounds containing natural amino acids with the L-configuration are preferred. Amino acids residues are abbreviated as follows throughout the application: Glycine is Gly or G; Valine is Val or V; Leucine is Leu or L; Glutamine is Gln or Q; Proline is Pro or P; Pyroglutamic acid (or pyrrolidone carboxylic acid) is Glp.

For the purpose of the present invention, the term "C-terminal" of a peptide is the end of the amino acid chain terminated by a free carboxyl group (-COOH). On the other hand, the term "N-terminal" refers to the end of a peptide terminated by an amino acid with a free amine group (-NH₂).

For the purpose of the present invention, the term "coupling" refers to the reaction between the carboxyl group of an amino acid or the C-terminus of a first peptide to the amino group of another amino acid or the N-terminus of a second peptide. In other words, during coupling, two peptide intermediate fragments, or a peptide intermediate fragment and a reactive amino acid, are coupled, generally, in an appropriate solvent, and usually in the presence of additional reagents that promote the efficiency and quality of the coupling reaction. The peptide intermediate fragments are reactively arranged so the N-terminus of one fragment becomes coupled to the C-terminus of the other fragment, or vice versa.

In a further particular aspect, the process according to the present invention comprises coupling a peptide of formula Val-Gln-Pro-Gly-Y (SEQ ID NO 2) with a peptide of formula X-Gly-Gly-Val-Leu (SEQ ID NO 3).

In the present invention, the protecting group is any sort of group that can prevents the atom or moiety to which it is attached, e.g., oxygen or nitrogen, from participating in undesired reactions during processing and synthesis. Protecting groups include side chain protecting groups and Cor N-terminal protecting groups. Protecting groups can also prevent reaction or bonding of carboxylic acids, thiols and the like.

The term "amino protecting group X" refers to protecting groups which can be used in the present invention to replace an acidic proton of an amino group in order to reduce its nucleophilicity. As it will be illustrated herein below, the amino protecting group X can be removed, if appropriate, in a deprotection reaction prior to possible subsequent addition of a next amino acid.

The amino protecting group X is preferably sterically hindering. The term "sterically hindering" is intended to denote in particular a substituent comprising at least 3 carbon atoms, in particular at least 4 carbon atoms, including at least one secondary, tertiary or quaternary carbon atom. The sterically hindering group often comprises at most 100, preferably at most 50 carbon atoms.

By way of non-limiting examples of suitable amino protecting groups represented herein by X, which can be used in the process according to the present invention, mention may in particular be made of substituted or unsubstituted groups of acyl type, such as the formyl, acrylyl (Acr), benzoyl (Bz), acetyl (Ac), trifluoroacetyl, substituted or unsubstituted groups of aralkyloxycarbonyl type, such as the benzyloxycarbonyl (Z), p-chlorobenzyloxycarbonyl, p-bromobenzyloxycarbonyl, p-nitrobenzyloxycarbonyl, p-methoxybenzyloxycarbonyl, benzhydryloxycarbonyl, 2-(p-biphenylyl)isopropyloxy-carbonyl, 2-(3,5-dimethoxyphenyl)isopropyloxycarbonyl, p-phenylazobenzyloxycarbonyl, triphenylphosphonoethyl-oxycarbonyl or 9-fluorenylmethyloxycarbonyl group (Fmoc), sub-stituted or unsubstituted groups of alkyloxycarbonyl type, such as the tert-butyloxycarbonyl (BOC), tert-amyloxycarbonyl, diisopropylmethyloxycarbonyl, iso-propyloxycarbonyl, ethyloxycarbonyl, allyloxycarbonyl, 2-methylsulphonylethyloxycarbonyl or 2,2,2-trichloro-ethyloxycarbonyl group, groups of cycloalkyloxycarbonyl type, such as the cyclopentyloxycarbonyl, cyclohexyl-oxycarbonyl, adamantyloxycarbonyl or isobornyloxy-carbonyl group, and groups containing a hetero atom, such as the benzenesulphonyl, p-toluenesulphonyl, mesitylenesulphonyl, methoxytrimethylphenyl-sulphonyl, 2- nitrobenzenesulfonyl, 2-nitrobenzenesulfenyl, 4- nitrobenzenesulfonyl or 4-nitrobenzenesulfenyl group. Among these groups X, those comprising a carbonyl, a sulfenyl or a sulphonyl group are preferred. An amino protecting group X is preferably selected from allyloxycarbonyl groups, tert-butyloxycarbonyl (BOC), benzyloxycarbonyl (Z), 9-fluorenylmethyloxycarbonyl (Fmoc), 4-nitrobenzenesulfonyl (Nosyl), 2-nitrobenzenesulfenyl (Nps) and substituted derivatives. More preferably, the amino protecting group X is tert-butyloxycarbonyl (BOC).

Amino protecting groups X may be introduced by various methods e.g. by reaction with suitable acid halides such as carbobenzoxyl chloride or acid anhydrides such as acetic anhydride. On the other hand, amino protecting groups X may be removed, for example, by acidolysis, hydrogenolysis, treatment with dilute ammonium hydroxide, treatment with sodium, treatment with sodium amide, treatment with hydrazine, or enzymatic hydrolysis.

The term "carboxylic acid protecting group Y" refers to protecting groups which can be used in the present invention to replace the acidic proton of a carboxylic acid. Preferred groups are selected from optionally substituted alkyl, aryl, aralkyl and, preferably, silyl groups. Trialkylsilyl groups are still more particularly preferred. Examples of such groups include methoxymethyl, methylthiomethyl, 2,2,2-trichloroethyl, 2-haloethyl, 2-(trimethylsilyl)ethyl, t-butyl, aryl, alkyl, aralkyl, allyl, benzyl, triphenylmethyl (trityl), benzhydryl, p-nitrobenzyl, p-methoxybenzyl, and trialkylsilyl groups such as trimethylsilyl, triethylsilyl, t-butyldimethylsilyl, i-propyl-dimethylsilyl. A trimethylsilyl group is more particularly preferred. In the process according to the present invention, the Val-Gln-Pro-Gly peptide is more preferably protected as a persilylated derivative. Good results were obtained by using MSA (N-Methyl-N-trimethylsilylacetamide). The persilylation of an amino acid or of a peptide can be carried out, for example, according to the method described in Patent Application EP 184243 B (SOLVAY) ))).

The carboxylic acid protecting groups Y may be introduced by various methods including esterification and silylation. On the other hand, the removal of carboxylic acid protecting groups Y may, for example, be effected by hydrolysis, saponification, acidolysis, hydrogenolysis or enzymatic hydrolysis.

It will be appreciated that the intermediate peptides, i.e. shorter peptides than the octapeptide having appropriate sequence of amino acids, to be coupled by the process according to the present invention may, if desired, be prepared using the solid-phase method of peptide synthesis. In such a method the carboxylic acid protecting group of the C-terminal amino acid is usually bonded to a resin.

In another particular aspect of the present invention, the process as above described may thus be carried out in the presence of a carboxylic acid activating agent.

For the purposes of the present invention, the term "carboxylic acid activating agent", also referred to as "coupling agent", is a reagent that replaces the hydroxyl group of a carboxylic acid with a suitable leaving group which is susceptible to nucleophilic displacement, allowing the coupling of an amino acid or peptide free carboxy group with a free amino group of another amino acid or peptide to form an amide bond between the reactants.

Examples of carboxylic acid activating agent and activated groups which are useful in the present invention include carbodiimides, acyl halides, azides, symmetric or mixed anhydrides, mixed anhydrides or active ester. Such carboxylic acid activating agent may be used before the coupling step in order to isolate the activated peptide derivative or used *in situ* prior to the introduction of the free amino peptide derivative.

Non-limitative particular examples of such carboxylic acid activating agent include carbodiimide reagents such as N,N'-dicyclohexylcarbodiimide (DCC), N-Ethyl-N'-(3-dimethylaminopropyl)carbodiimide (EDC), 1-(3-dimethylaminopropyl)-3-ethylcarbodiimide hydrochloride (EDCI also referred to as "WSC", carbodiimidazoles reagents such as 1,1'-carbonyldiimidazole (CDI), ), diisopropylcarbodiimide (DIPCDI), diisopropylcarbodiimide (DIC) or derivatives thereof; phosphonium salts such as (benzotriazol-1-yloxy)tris-(dimethylamino)phosphonium (BOP), benzotriazol-1-yl-oxytripyrrolidinophosphonium hexafluorophosphate (PyBOP), (7-Azabenzotriazol-1-yloxy)tripyrrolidinophosphonium hexafluorophosphate (PyAOP), bromo-tris-pyrrolidino phosphoniumhexafluorophosphate (PyBroP), chloro-tris-pyrrolidino phosphoniumhexafluorophosphate (PyCloP) or derivatives thereof; uronium or guanidinium salts such as o-benzotriazole-N,N,N',N'-tetramethyl-uronium-hexafluoro-phosphate (HBTU), o-(benzotriazol-1-yl)-N,N,N',N'-tetramethyluronium tetrafluoroborate (TBTU),
2-(7-aza-1H-benzotriazole-1-yl)-1,1,3,3-tetramethyluronium hexafluorophosphate (HATU), O-(7-azabenzotriazol-1-yl)-1,1,3,3-bis(tetramethylene)uronium hexafluorophosphate (HAPyU) or derivatives thereof; acyl halides such as isobutyl chloroformate (iBCF), pivaloyl chloride (PivCl), t-butylchloroformate (TBCF), ethyl chloroformate (ECF) or derivatives thereof; esterificating agent such as pentafluorophenol (PfP), N-hydroxysuccinimide (NHS) or derivatives thereof; azidination agent such as diphenylphosphoryl azide (DPPA) or derivatives thereof. Preactivated amino acids or under the form of N-carboxyanhydrides, and in particular urethane-N-carboxyanhydrides (UNCA's) are also good examples of carboxylic acid activating agents.

The carboxylic acid activating agent is preferably chosen from carbodiimides, carbonyldiimidazoles, acyl halides, phosphonium salts and uronium or guanidinium salts and more preferably from isobutyl chloroformate and pivaloylchloride.

Good results are often obtained when using additional reagents which reduce side reactions and/or increase reaction efficiency. For example, phosphonium and uronium salts can, in the presence of a tertiary base, for example, diisopropylethylamine (DIPEA) and triethylamine (TEA), convert protected amino acids into activated species (for example, BOP, PyBOP, HBTU, and TBTU all generate HOBt esters). Other reagents which help prevent racemization include carbodiimides (for example, DCC or WSCDI) with an added auxiliary nucleophile (for example, 1-hydroxy-benzotriazole (HOBt), 3,4-dihydro-3-hydroxy-4-oxo-1,2,3-benzotriazine (HOOBT), 1-hydroxy- azabenzotriazole (HOAt), or HOSu) or derivatives thereof. Another reagent that can be utilized is TBTU. The mixed anhydride method, using isobutyl chloroformate, with or without an added auxiliary nucleophile, is also used, as is the azide method, due to the low racemization obtained. These types of compounds can also increase the rate of carbodiimide- mediated couplings, as well as prevent dehydration of Asn and Gln residues.

When such carboxylic acid activating agents are used, the coupling reaction is often carried out in the presence of a base as additional reagent. In another particular aspect of the present invention, the coupling reaction is thus carried out in the presence of a base. The base is preferably chosen from tertiary and heteroaromatic amines such as N-methylmorpholine (NMM), pyridine, triethylamine (TEA), diisopropylethylamine (DIPEA) or mixtures thereof. More preferably, it is N-methylmorpholine.

In another particular aspect of the present invention, the peptide coupling as above described is carried out in a polar organic solvent. In a particular preferred embodiment, the polar organic solvent allows for particularly efficient control of racemization of the peptide bond formed, the solubility of the peptide and/or peptide fragments, and the coupling reaction rate. The polar organic solvent is preferably selected from N,N-dimethylacetamide (DMA), N,N-dimethylformamide (DMF), N-methylpyrrolidone (NMP), dimethylsulfoxide (DMSO), ethyl acetate (AcOEt), dichloromethane (DCM), methylene chloride, pyridine, chloroform, acetonitrile, dimethoxyethane, dioxane, tetrahydrofuran (THF) or mixtures thereof. More preferably, it is selected from N,N-dimethylacetamide (DMA), N-methylpyrrolidone (NMP) and N, N-dimethylformamide (DMF). The most preferably, the polar organic solvent is N,N-dimethylacetamide (DMA).

In the present invention, the coupling reaction is generally carried out at a temperature of greater than or equal to -45°C. Often, the reaction is carried out at a temperature greater than or equal to -25°C. Preferably, the temperature is greater than or equal to -20°C. In the process according to the invention, the reaction is generally carried out at a temperature of less than or equal to +45°C. Often, the reaction is carried out at a temperature of less than or equal to +5°C. Preferably, the temperature is less than or equal to 0°C.

In another particular aspect of the present invention, the solution can suitably be treated after the coupling step with an aqueous phase so as to provide a solution of coupled product in the aqueous phase and then, the coupled product is extracted from the aqueous phase into an organic solvent.

In this case, the pH value of the aqueous phase is preferably controlled to be greater than or equal to 1. More preferably, it is greater than or equal to 1.5. Still more preferably, it is greater than or equal to 2. On the other hand, the pH value of the aqueous phase is preferably controlled to be less than or equal to 5. More preferably, it is less than or equal to 3.5. Still more preferably, it is less than or equal to 3. Excellent results were obtained with a pH value of the aqueous phase of about 2.5.

Usually, the reaction product after the coupling step contains one or more protecting group(s). An example of such coupling product is a peptide derivative of formula X-Gly-Gly-Val-Leu-Val-Gln-Pro-Gly-Y, wherein X and Y are as defined above. If desirable, the protecting groups can be removed, for example in a selective way. Thus it is possible to remove only certain protecting groups, keeping others intact during the subsequent reaction(s).

In a preferred aspect of the process according to the invention, the peptide derivative of formula X-Gly-Gly-Val-Leu-Val-Gln-Pro-Gly-Y is further deprotected of the amino protecting group X and of the acid protecting group Y to provide the free Gly-Gly-Val-Leu-Val-Gln-Pro-Gly octapeptide (SEQ ID NO 1).

In the process according to the invention, at least one peptide coupling step is carried out in solution. Preferably, at least 2, for example 2, 3 or 4 and more preferably at least 5 peptide coupling steps for example 5, 6 or 7 coupling steps are carried out in solution. Still more preferably, all coupling steps are carried out in solution. Particular solution phase coupling steps which are useful in the process according to the invention are apparent from the synthesis in the scheme hereafter.

Gly Gly Val Leu Val Gln Pro Gly
1 2 3 4 5 6 7 8
X-Glp-OH
X OH
X Y H OH X OH
X OH H OH X Y H-Gln OH
X OH H OH
H OH

Good results were obtained when the fragments 3-4, 6-7, 6-8 and 5-8 of scheme 1 were under their persilylated form.

In the process according to the invention, the pressure in the solution phase coupling step is generally chosen so as to maintain the solution in the liquid state. Atmospheric pressure (approximately 101.3 kPa) and superatmospheric pressures are very suitable.

The reaction products can be isolated and purified by purification methods, such as for example extraction, crystallization, precipitation or chromatography (e.g. thin layer or column). Intermediates and the end products may, for example, be characterized by chromatographic parameters (purity control), optical rotation and possibly spectroscopic data.

### Synthesis of X-Val-GIn-Pro-Gly peptide (SEQ ID NO 2)

In still another particular aspect of the present invention, the process as above described may comprise the manufacture of X-Val-Gln-Pro-Gly peptide (SEQ ID NO 2) wherein X is an amino protecting group as described above by coupling of X-Val with Gln-Pro-Gly peptide.

Such tetrapeptide (SEQ ID NO 2) manufacture preferably comprises activating the carboxylic acid function of X-Val e.g. in an activated ester form of the X-Val, preferably with N-hydroxysuccinimide. In particular, X may be benzyloxycarbonyl (Z).

The coupling between the X-Val and the Gln-Pro-Gly peptide is preferably carried out in solution. In this case, the pressure is chosen so as to maintain the solution in the liquid state. Atmospheric pressure (approximately 101.3 kPa) and superatmospheric pressures are very suitable. When this coupling according to the present invention is carried out in solution, said solution generally comprises acetonitrile (CH₃CN) and/or an aqueous medium.

The coupling between the X-Val and the Gln-Pro-Gly peptide is generally carried out at a temperature of greater than or equal to -30°C. Often, the reaction is carried out at a temperature greater than or equal to -10°C. Preferably, the temperature is greater than or equal to -5°C.This reaction is generally carried out at a temperature of less than or equal to +50°C. Often, the reaction is carried out at a temperature of less than or equal to +30°C. Preferably, the temperature is less than or equal to +25°C.

The reaction medium may then be suitably treated after the coupling step with an aqueous phase so as to provide a solution of coupled product in the aqueous phase and then, the X-Val-Gln-Pro-Gly peptide (SEQ ID NO 2) is extracted from the aqueous phase into an organic solvent.

The X-Val-Gln-Pro-Gly peptide (SEQ ID NO 2) may then be recovered by precipitation. In that case, the X-Val-Gln-Pro-Gly peptide (SEQ ID N02) is generally provided as solution in a first solvent and then precipitated by the addition of a second solvent wherein the peptide is less soluble than in the first solvent.

The second solvent advantageously comprises at least one solvent chosen from diisopropyl ether, acetonitrile, diethyl ether, methylterbutylether, ethyl acetate, isopropyl acetate, acetone, tetrahydrofuran, dichloromethane or dioxane. Good results were obtained with diisopropyl ether.

### Synthesis of Val-Gln-Pro-Gly tetrapeptide (SEQ ID NO 2)

In still another particular aspect of the present invention, the process as above described may comprise the manufacture of Val-Gln-Pro-Gly tetrapeptide (SEQ ID NO 2), wherein the manufacture of the X-Val-Gln-Pro-Gly peptide as above described further comprises the deprotection of the N-terminal amino protecting group X. In particular, X may be benzyloxycarbonyl (Z).

Such amino protecting groups X may be removed by acidolysis, hydrogenolysis, treatment with dilute ammonium hydroxide, treatment with sodium, treatment with sodium amide, treatment with hydrazine, or enzymatic hydrolysis. It is preferably removed by hydrogenolysis.

### Synthesis of X-Gln-Pro-Gly tripeptide

In still another particular aspect of the present invention, the process as above described comprises in addition the manufacture of X-Gln-Pro-Gly tripeptide, wherein X is an amino protecting group, by ring opening of X-Glp-Pro-Gly tripeptide with ammonia.

### Synthesis of Gly-Gly-Val-Leu tetrapeptide (SEQ ID NO 31

In still another particular aspect of the present invention, the process as above described comprises in addition the manufacture of Gly-Gly-Val-Leu tetrapeptide (SEQ ID NO 3) by coupling of Val-Leu dipeptide with X-Gly-Gly or X-Gly.

Generally, this coupling is carried out in the presence of a carboxylic acid activating agent. Carbodiimides, acyl halides, phosphonium salts and uronium or guanidinium salts are generally preferred as carboxylic acid activating agent. More preferably, the carboxylic acid activating agent is an acyl halide. Still more preferably, it is chosen from isobutyl chloroformate and pivaloylchloride.

When such carboxylic acid activating agents are used, this coupling is generally carried out in the presence of a base as additional reagent. It is preferably chosen from N-methylmorpholine (NMM), pyridine, diisopropylethylamine (DIPEA) or triethylamine (TEA). More preferably it is N-methylmorpholine (NMM).

The coupling of a Val-Leu dipeptide with X-Gly-Gly or X-Gly is preferably carried out in solution. In this case, the pressure is chosen so as to maintain the solution in the liquid state. Atmospheric pressure (approximately 101.3 kPa) and superatmospheric pressures are very suitable. The solution generally comprises a polar organic solvent. Preferably, the polar organic solvent is selected from N,N-dimethylacetamide, N,N-dimethylformamide, N-methylpyrrolidone , dimethylsulfoxide, ethyl acetate, dichloromethane or mixtures thereof. More preferably, the solution comprises N,N-dimethylacetamide or ethyl acetate.

The Glycine amino acid or dipeptide Gly-Gly is protected by an amino protecting group X. The amino protecting group X is preferably selected from tert-butyloxycarbonyl, benzyloxycarbonyl, 9-fluorenylmethyloxycarbonyl, 2-nitrobenzenesulfonyl, 2-nitrobenzenesulfenyl, and substituted derivatives. More preferably, the amino protecting group X is tert-butyloxycarbonyl (BOC).

The Val-Leu dipeptide is generally protected by a carboxylic acid protecting group Y.

Preferred carboxylic acid protecting group Y are alkyl, aryl and silylated derivatives. In a particularly preferred variant, the Val-Leu peptide derivative is a persilylated derivative. The persilylation of Val-Leu dipeptide can be carried out, for example, according to the method described in Patent Application EP-A-184243 in the Applicant's name. It is preferably carried out with MSA.

The coupling of a Val-Leu dipeptide with a X-Gly-Gly or X-Gly is generally carried out at a temperature of greater than or equal to -45°C. Often, the reaction is carried out at a temperature greater than or equal to -25°C. Preferably, the temperature is greater than or equal to -20°C. In the method according to the invention, the coupling is generally carried out at a temperature of less than or equal to +45°C. Often, this reaction is carried out at a temperature of less than or equal to +5°C. Preferably, the temperature is less than or equal to 0°C.

The tetrapeptide obtained as above described may be further deprotected of the amino protecting group X and of the acid protecting group Y to provide the free Gly-Gly-Val-Leu tetrapeptide (SEQ ID NO 3).

The reaction products can then be isolated and purified by purification methods, such as for example extraction, crystallization, precipitation or chromatography (e.g. thin layer or column).

The coupling steps as above described of the present invention may be carried out under persilylation conditions. In other words, the amino acids or peptides used in the process according to the present invention may be protected under their persilylated form. They are preferably protected under their persilylated form.

Another aspect of the present invention is related to a tripeptide of the formula Glp-Pro-Gly or protected peptide of the formula X-Glp-Pro-Gly, wherein X is an amino protecting group, to a tripeptide of the formula Gln-Pro-Gly or protected peptide of the formula X-Gln-Pro-Gly, wherein X is an amino protecting group, to a tetrapeptide of the formula Val-Gln-Pro-Gly (SEQ ID NO 2) or protected peptide of the formula X-Val-Gln-Pro-Gly (SEQ ID NO 2), wherein X is an amino protecting group and in particular when X is benzyloxycarbonyl, to a pentapeptide of the formula Leu-Val-Gln-Pro-Gly (SEQ ID NO 4), to an hexapeptide of the formula Val-Leu-Val-Gln-Pro-Gly (SEQ ID NO 5) and to an heptapeptide of the formula Gly-Val-Leu-Val-Gln-Pro-Gly (SEQ ID NO 6), which may be obtained, as such or under their protected form, as intermediates during a process according to the present invention.

Besides, the present invention also relates to a dodecapeptide of the formula Gly-Gly-Val-Leu-Val-Gln-Pro-Gly-Val-Gln-Pro-Gly (SEQ ID NO 7), to an hexakaidecapeptide of the formula Gly-Gly-Val-Leu-Val-Gln-Pro-Gly-Gly-Gly-Val-Leu-Val-Gln-Pro-Gly (SEQ ID NO 8) and to a modified peptide of the formula CH₃ C(=O)-NH-Gly-Gly-Val-Leu-Val-Gln-Pro-Gly (SEQ ID NO 1), which may be obtained during a process according to the present invention.

The present invention also relates to the use of the following peptides as intermediates in peptide synthesis:
Tripeptide of the formula Glp-Pro-Gly;
Tetrapeptide of the formula Val-Gln-Pro-Gly (SEQ ID NO 2);
pentapeptide of the formula Leu-Val-Gln-Pro-Gly (SEQ ID NO 4);
hexapeptide of the formula Val-Leu-Val-Gln-Pro-Gly (SEQ ID NO 5);
heptapeptide of the formula Gly-Val-Leu-Val-Gln-Pro-Gly (SEQ ID NO 6),
as such or under their protected form.

### Purification

One of the major issues in peptide synthesis is related to the isolation and purification of the peptides, which are often the cause of a reduction in the yield of the final peptide product.

In consequence, the invention also relates to the purification of the above described peptides, as such or under their protected form. The purification according to the invention allows in particular meeting specifications concerning purification related to organic impurities such as for example organic solvents, such as acetonitrile and is industrializable.

The purification process according to the present invention allows an efficient and low cost production of said purified octapeptide.

It is thus also an object of the present invention to provide a process for the purification of Gly-Gly-Val-Leu-Val-Gln-Pro-Gly peptide (SEQ ID NO 1) or anyone of the peptides of the formula Val-Gln-Pro-Gly (SEQ ID NO 2) or X-Val-Gln-Pro-Gly (SEQ ID NO 2), wherein X is an amino protecting group, Leu-Val-Gln-Pro-Gly (SEQ ID NO 4), Val-Leu-Val-Gln-Pro-Gly (SEQ ID NO 5), Gly-Val-Leu-Val-Gln-Pro-Gly (SEQ ID NO 6), Gly-Gly-Val-Leu-Val-Gln-Pro-Gly-Val-Gln-Pro-Gly (SEQ ID NO 7), Gly-Gly-Val-Leu-Val-Gln-Pro-Gly-Gly-Gly-Val-Leu-Val-Gln-Pro-Gly (SEQ ID NO 8), and CH₃C(=O)-NH-Gly-Gly-Val-Leu-Val-Gln-Pro-Gly (SEQ ID NO 1), as such or under a protected form wherein the peptide is dissolved in a first solvent and then precipitated or crystallized by the addition of a second solvent wherein the peptide is less soluble than in the first solvent.

The peptide may be dissolved by the addition of a first solvent or may be directly obtained after the work-up as a solution in a first solvent. In this latest case, the solution may be concentrated under vacuum before the addition of the second solvent.

The nature of the first and second solvent depends on the nature of the peptide, on its isoelectric point value and on its protected or unprotected form. Typically, when the peptide is unprotected, the first solvent is preferably an aqueous medium and the second solvent preferably comprises at least one polar organic solvent. The pH of the aqueous medium may in some cases preferably be controlled. On the other hand, in the case of a protected peptide, the first solvent comprises preferably at least one polar organic solvent while the second solvent is preferably an aqueous medium.

The polar organic solvent is preferably chosen from isopropyl ether (IPE), acetonitrile (CH₃CN), methylterbutylether (MTBE), ethyl acetate (AcOEt), isopropyl acetate (AcOiPr), acetone, tetrahydrofurane (THF), dichloromethane (DCM), dioxane, methanol, tert-butanol, isopropanol, acetic acid, N,N-dimethylacetamide (DMA), N,N-dimethylformamide (DMF) and the like or mixtures thereof. Good results were obtained with isopropyl ether and/or acetonitrile.

The table hereafter gives preferred combinations of first and second solvents for the different fragments.

**Table 1**

| ***Peptide*** | ***First solvent*** | ***Second solvent*** |
|---|---|---|
| H-Val-Leu-OH | methanol / water | isopropanol |
| Z-Glp-Pro-OH | water / acetonitrile | water |
| Z-Glp-Pro-Gly-OH | Water/ dichloromethane | Aqueous KHSO₄ solution |
| H-Gln-Pro-Gly-OH | water | ethanol |
| Z-Val-Gln-Pro-Gly-OH (SEQ ID NO 2) | Isobutanol / dichloromethane | diisopropylether |
| H-Val-Gln-Pro-Gly-OH (SEQ ID NO 2) | methanol | acetonitrile |
| HCl.H-Gly-Gly-Val-Leu-Val-Gln-Pro-Gly-O H (SEQ ID NO 1) | Acetic acid / dioxane | diisopropylether |
| CH₃COO-^{.} H-Gly-Gly-Val-Leu-Val-Gln-Pro-Gly-OH (SEQ ID NO 1) | Ammonium acetate aqueous buffer | acetonitrile |

It may be advantageous to isolate and purify the desired peptide product by salt formation (e.g. hydrochloride, acetate, dicyclohexyl ammonium, cyclohexyl ammonium or trifluoroacetate salt formation).

The peptide can also be separated from a solution for example by spray-drying, filtration or decantation and dried before optionally being submitted to further processing steps such as combining with other ingredients, lyophilization, spray-drying, packaging and/or storage.

According to one suitable approach, the peptide is collected via filtering and optionally washed, in particular to reduce possible salt content, and then dried.

A further particular aspect of the present invention is related to a process for purifying Gly-Gly-Val-Leu-Val-Gln-Pro-Gly peptide (SEQ ID NO 1), or anyone of the peptides of the formula Val-Gln-Pro-Gly (SEQ ID NO 2) or X-Val-Gln-Pro-Gly (SEQ ID NO 2), wherein X is an amino protecting group, Leu-Val-Gln-Pro-Gly (SEQ ID NO 4), Val-Leu-Val-Gln-Pro-Gly (SEQ ID NO 5), Gly-Val-Leu-Val-Gln-Pro-Gly (SEQ ID NO 6), Gly-Gly-Val-Leu-Val-Gln-Pro-Gly-Val-Gln-Pro-Gly (SEQ ID NO 7), Gly-Gly-Val-Leu-Val-Gln-Pro-Gly-Gly-Gly-Val-Leu-Val-Gln-Pro-Gly (SEQ ID NO 8), and CH₃C(=O)-NH-Gly-Gly-Val-Leu-Val-Gln-Pro-Gly (SEQ ID NO 1) as such or under a protected form, which comprises subjecting a crude peptide of any of the aforesaid sequences to a chromatography operation.

In the present invention, the chromatography is preferably chosen from medium pressure liquid chromatography (MPLC) and high pressure liquid chromatography (HPLC).

In this aspect, the chromatography operation may take place prior or after an optional precipitation or crystallisation of the peptide.

The chromatography operation may be processed for example on columns with a continuous bed (monolithic columns). In that case, normal phase stationary phases can be used, for example silica or alumina. In that case apolar mobile phases are generally used. Reverse phase stationary phases such as hydrophobically modified inorganic supports, typically silica grafted with organic hydrophobic compounds are preferably used. In that case polar mobile phases are generally used, for example aqueous mobile phases containing an organic co-solvent, in particular a polar organic co-solvent, such as methanol, ethanol, isopropanol, acetonitrile or dioxane. Medium polar such as silica with bonded diol, propylcyano or amino groups may also be used in particular with moderately polar mobile phases such as buffered aqueous / organic mobile phases.

The chromatography operation may be a medium pressure liquid chromatography (MPLC). In such a chromatography operation, the eluent may comprise water, acetonitrile (CH₃CN), alcohols such as methanol, ethanol, propanol and the like. Preferably, it comprises water (H₂O) and/or acetonitrile (CH₃CN). The eluent may also comprise a certain amount of salts to maintain its pH value to a certain area (buffer solution). Good results were obtained when an aqueous solution of ammonium acetate was used as eluent.

A further particular aspect of the present invention is related to a solution of Gly-Gly-Val-Leu-Val-Gln-Pro-Gly peptide (SEQ ID NO 1) or anyone of the peptides of the formula Val-Gln-Pro-Gly (SEQ ID NO 2) or X-Val-Gln-Pro-Gly (SEQ ID NO 2), wherein X is an amino protecting group, Leu-Val-Gln-Pro-Gly (SEQ ID NO 4), Val-Leu-Val-Gln-Pro-Gly (SEQ ID NO 5), Gly-Val-Leu-Val-Gln-Pro-Gly (SEQ ID NO 6), Gly-Gly-Val-Leu-Val-Gln-Pro-Gly-Val-Gln-Pro-Gly (SEQ ID NO 7), Gly-Gly-Val-Leu-Val-Gln-Pro-Gly-Gly-Gly-Val-Leu-Val-Gln-Pro-Gly (SEQ ID NO 8), and CH₃C(=O)-NH-Gly-Gly-Val-Leu-Val-Gln-Pro-Gly (SEQ ID NO 1), as such or under a protected form, in a solvent mixture comprising water and a polar organic solvent.

The above described solution according to the invention preferably comprises acetonitrile and alcohols such as methanol, ethanol, propanol and the like. Such solution may be used in a purification operation.

Another aspect of the present invention is relative to a process for the isolation of the Gly-Gly-Val-Leu-Val-Gln-Pro-Gly octapeptide (SEQ ID NO 1) from an aqueous medium wherein the aqueous medium containing the peptide is lyophilized. A lyophilization is intended to denote a means of drying a desired substance, achieved by freezing an aqueous medium containing said substance and causing ice to sublime directly to vapor by exposing it to a low partial pressure of water vapor.

The following examples are intended to illustrate the invention without however limiting its scope.

### EXAMPLES

The following examples are intended to illustrate the invention without, however, limiting its scope.

In these examples and throughout this specification the abbreviations employed are defined as follows:

AcOH is acetic acid, AcOEt is ethyl acetate, Boc is t-butoxycarbonyl, n-BuOH is n-butanol, i-BuOH is iso-butanol, Cbz is benzyloxycarbonyl, DCC is 1,3 dicyclohexylcarbodiimide, DCM is dichloromethane, DIC is 1,3-diisopropylcarbodiimide, DIPEA is N,N-diisopropylethylamine, DMF is N,N-dimethylformamide, DMA is N,N-dimethylacetamide, Fmoc is fluorenylmethyloxycarbonyl, HBTU is N,N,N',N'-tetramethyl-O-(1H-benzotriazol-1-yl)uronium-hexafluororphosph ate), HOBT is 1-hydroxybenzotriazole, HOOBT is 3,4-dihydro-3-hydroxy-4-oxo-1,2,3-benzotriazine, IBCF is isobutyl chloroformate, i-BuOH is isobutanol, IPE is diisopropylether, MeCN is acetonitrile, MeOH is methanol, NMM is N-methylmorpholine, NMP is 1-methyl-2-pyrrolidone, THF is tetrahydrofuran, MSA is N-Methyl-N-trimethylsilylacetamide, Tos is tosyl,

The following scheme represents the general synthetic approach of the Gly-Gly-Val-Leu-Val-Gln-Pro-Gly (SEQ ID NO 1) octapeptide which will be detailed in the following examples.

Gly Gly Val Leu Val Gln Pro Gly
1 2 3 4 5 6 7 8
Z - Glp-OH
Z OSu
Z OH
Z OH
Z Gln OH
Z OSu H OH Z OSu H OH
Boc OH H OH Z OH
Boc OH H OH
Boc OH
H OH

### Example 1: synthesis of H-Val-Leu-OH

Leucine (1.2 eq.) was silylated in pure MSA at 50°C until complete dissolution and then diluted with AcOEt. The leucine solution was transferred to a Z-Val-OSu solution under stirring at 35°C. The reaction was quenched with water, diluted with AcOEt and the organic phase was washed with KHSO₄ and NaCl. The solvent was removed under vacuum and replaced with MeOH. Water was then added to the methanolic solution followed by the addition of the palladium catalyst. The deprotection of the Z group took place with the introduction of gaseous hydrogen at 30°C. Once the reaction completed, the catalyst was filtered and washed with a 50/50 mixture of methanol and water. The solvent was evaporated and the mixture diluted with isopropanol to precipitate the dipeptide. The dipeptide was then recovered by filtration, washed with isopropanol at room temperature and then dried. The dipeptide was isolated with a yield of 85%.

### Example 2: synthesis of Boc-Gly-Gly-Val-Leu-OH (SEQ ID NO 3)

On one hand, H-Val-Leu-OH (1 eq.) was added to a solution of MSA (2.72 eq.) in AcOEt. The slurry was stirred at 25°C until a solution was obtained. The solution was then cooled to -15°C. On the other hand, Boc-Gly-Gly-OH (1.05 eq., commercially available) was added together with NMM (1.0 eq.), AcOEt and DMF. The slurry was stirred until complete dissolution and then cooled to -25°C. IBCF (1.0 eq.) was added to the Boc-Gly-Gly-OH solution to activate the carboxylic function. The silylated Val-Leu was then added and left to stir at least 30 min. The reaction mixture was conditioned to 25°C before being quenched by the addition of water. The mixture was then diluted with AcOEt and washed with a solution of KHSO₄ under stirring. The aqueous phase was discarded and the organic layer was washed again with a solution of NaCl. The organic phase was finally concentrated and the tetrapeptide crystallized under gentle stirring for at least 8h at 5°C. The solid was recovered by filtration, once washed with cold AcOEt at 5°C. After drying under vacuum, 85 % of Boc-Gly-Gly-Val-Leu-OH (SEQ ID NO 3) was recovered.

### Example 3: synthesis of Z-Glp-Pro-OH

Z-Glp-OH.DCHA (commercially available, 1 eq.) was diluted in AcOEt and neutralized by the addition of an aqueous solution of KHSO₄. The organic phase was collected and the aqueous phase was extracted with another volume of AcOEt. The combined organic phases were then washed with water and the solvent (AcOEt) was replaced with MeCN. Suc-OH (1.05 eq.) was dissolved in the solution of ZGIpOH which was then cooled to -5°C. DCC (1.1 eq.) dissolved in MeCN was added slowly to the solution keeping the reaction temperature below 5°C. The reaction was allowed to warm to 25°C over at least 4h. The excess of DCC was neutralized with AcOH (0.05 eq.) and the suspension was cooled to 10°C before filtering the DCU precipitated which was then washed with MeCN. The resulting solution was warmed to 25°C. H-Pro-OH (2.0 eq.) was added to a solution of MeCN and (1.9 eq.) of MSA. The suspension was heated to 45°C and left stirring until a clear solution was obtained. The solution was then cooled to 25°C. The silylated proline solution was added to the solution of Z-Glp-OSu and left to stir for at least 3 h at 25°C. The coupling solution was diluted with water and MeCN was evaporated in vacuo at a maximum temperature of 65°C. The remaining slurry was then diluted with water and the precipitate was left stirring for at least 10h at 5°C. The solid was filtered, washed with water. After drying under vacuum 80 % of Z-Glp-Pro-OH was recovered.

### Example 4: synthesis of Z-Glp-Pro-Gly-OH

Two solutions were prepared before the peptide coupling. Solution A : H-Gly-OH (1.2 eq.) was dissolved in MSA (3.0 eq.) at maximum 60°C. The suspension was cooled down to 25°C, diluted with DCM and stirred for at least 8 hours before being cooled to -15°C. In solution B, the carboxylic function of the Z-Glp-Pro-OH was dissolved with DCM and NMM (1.05 eq.). The solution was cooled to -15°C. The carboxylic acid was activated with IBCF (1.05 eq.) and the silylated solution A was then added to the slurry. The slurry was stirred for at least 0.5h and left to warm to 25°C. The mixture was quenched with water and the addition of a solution of KHSO₄ under stirring precipitated the peptide.. The solid was filtered and washed with water. After drying under vacuum, 80 % of Z-Glp-Pro-Gly-OH was recovered.

### Example 5: synthesis of H-Gln-Pro-Gly-OH

Z-Glp-Pro-Gly-OH (1 eq.) was dissolved under stirring in DMA at 25°C and NH₄OH 25% (6 eq.) was added in such way that the temperature did not rise above 30°C. The mixture was stirred for at least 4h at 25°C. The solution was concentrated under vacuum till the pH was ≤ 3. The concentrate was diluted with a NaCl aqueous solution and the pH was adjusted to 2.5 with a solution of KHSO₄. The resulting aqueous solution was washed twice with IPE and then extracted three times with n-BuOH at 25°C. The organic layers were combined and washed with water. The resulting organic solution was concentrated under reduced pressure. The concentrated solution was diluted with ethanol and water at 20°C and Pd/C (0.02 eq.) was added to the peptide solution. The solution was stirred at 20°C followed by the introduction of hydrogen under pressure (0.3 bar). The solution was stirred for at least 2 hours and the completion of the reaction was checked by HPLC. The solution was filtered to remove the catalyst and, after one washing with demineralised water, the pH of the solution was adjusted to 6.0 ≤ pH ≤ 6.5 with an aqueous solution of NaHCO₃. The free tripeptide was then precipitated by addition of ethanol. The solution was left to mature at 25°C for 3 hours. The solid was filtered and washed with ethanol. After drying under vacuum, 60 % of H-Gln-Pro-Gly-OH was recovered.

### Example 6: synthesis of H-Val-Gln-Pro-Gly-OH (SEQ ID NO 2)

H-Gln-Pro-Gly-OH (1 eq.) was added to H₂O containing DIPEA (2.00 eq.). The slurry was stirred at 25°C until a clear solution was observed and was then cooled to 0°C. Z-Val-OSu (1.1 eq.) was dissolved in MeCN at 25°C until a clear solution was obtained and was then cooled to 0°C. The Z-Val-OSu solution was added to the solution of H-Gln-Pro-Gly-OH (SEQ ID NO 2) in such way that the temperature did not rise above 5°C. Then the mixture was stirred for at least 2h. The peptide solution was concentrated and then diluted with a KHSO₄ solution and stirred for a few minutes. This aqueous solution was washed twice with a mixture of IPE and AcOEt. The organic phases were discarded and the aqueous phase was extracted 2 times with a 20% i-BuOH in DCM solution. The organic phases were collected and concentrated under reduced pressure until water content in the evaporates was ≤ 1 % weight. The solution of the protected tetrapetide was then precipitated in IPE at 25°C. Z-Val-Gln-Pro-Gly-OH (SEQ ID NO 2) was collected by filtration, washed with IPE and dried under vacuum until IPE content in the peptide was ≤ 5% weight. The protected fragment was dissolved in methanol at 30°C and Pd/C (0.02 eq.) was added to the peptide solution. The solution was stirred at 20°C followed by the introduction of hydrogen under pressure (0.3 bar). After stirring for 3 hours the solution was filtered to remove the catalyst which was washed with methanol. After evaporation, the free tetrapeptide was then precipitated by transferring the solution into MeCN at 10°C. The solid was filtered and washed with MeCN. After drying under vacuum, 60 % of H-Val-Gln-Pro-Gly-OH (SEQ ID NO 2) was recovered.

### Example 7: synthesis of H-Gly-Gly-Val-Leu-Val-Gln-Pro-Gly-OH (SEQ ID NO 1)

H-Val-Gln-Pro-Gly-OH (SEQ ID NO 2) (1.0 eq.) was silylated by adding it to a solution of DMA containing MSA (3.2 eq.) at a temperature ≤40°C until a clear solution was observed. This solution was then cooled to -15°C. Boc-Gly-Gly-Val-Leu-OH (1.0 eq. (SEQ ID NO 3)) was dissolved in DMA with NMM (1.0 eq.) until a clear solution was obtained. The solution was cooled to -25°C. IBCF (1.0 eq.) was added to the solution of Boc-Gly-Gly-Val-Leu-OH (SEQ ID NO 3) to activate the acid function. The solution of the activated tetrapeptide was then transferred as quickly as possible to the solution of the silylated H-Val-Gln-Pro-Gly-OH (SEQ ID NO 2). The reaction medium was stirred for at least 0.5 h and left to warm to -5°C. The reaction mixture was neutralized at -5°C by addition of 5% KHSO₄ and was then concentrated under reduced pressure. This solution was diluted successively with water and i-BuOH. The pH was adjusted to 2.5 by the controlled addition of a 5% aqueous KHSO₄ solution, and then DCM was introduced to extract the octapeptide. The aqueous solution was discarded and the organic phase was once washed with a solution of NaCl. The organic phase was then concentrated under reduced pressure and the solvent was replaced with glacial AcOH until water content was ≤2% weight and i-BuOH content ≤2% weight in the evaporates. To remove the Boc group, HCl 4M in dioxane (5 eq.) was added and the mixture was stirred at 45°C for 2 h. The final peptide was recovered by precipitation in IPE at 25°C. The solid was filtered, once washed with IPE and once with MeCN. After drying under vacuum at 40°C, 80% of HCl.H-Gly-Gly-val-Leu-Val-Gln-Pro-Gly-OH (SEQ ID NO 1) was recovered. The peptide was solubilised in a 0.05M ammonium acetate buffer solution at 25°C, adjusted to 4.5 ≤ pH ≤ 5.0 with a 25% NH₃ solution, and then diluted with MeCN. This solution was filtered and purified as described in example 8.

### Example 8: purification of H-Gly-Gly-Val-Leu-Val-Gln-Pro-Gly-OH (SEQ ID NO 1)

The chromatography was operated on an Amberchrom CG161m, using as mobile phases : A = "aqueous" : 0.05 M NH₄OAc (pH ∼7.5) and B= "organic" : 0.05 NH₄OAc in 50/50 HPW/MeCN. The stationary phase was first conditioned with a 10% solution of B, the crude product obtained in example 7 was then injected to the stationary phase and washed with a 10% solution of B. The eluent was then added (22 % B) and the stationary phase was then washed with a 100% solution of B.

### Example 9: freeze-drying of H-Gly-Gly-Val-Leu-Val-Gln-Pro-Gly-OH (SEQ ID NO 1)

The solution of the peptide obtained in example 8 was concentrated under vacuum and lyophilized in GORE^{™} LYOGUARD^{®}freeze-drying trays. The peptide solution was placed into a freeze-dryer (GT4 Edwards/Kniese) for lyophilisation. The freeze-drying trays were cooled to -40°C for 3 h, then the temperature was raised to 20°C under vacuum (0,22 mbar) for 17 h. After finishing main drying the temperature was maintained to 20°C for 4 h with a vacuum adjusted to 0,02 mbar. A white powder of H-Gly-Gly-Val-Leu-Val-Gln-Pro-Gly-OH (SEQ ID NO 1) was obtained.

### Sequence Listing Free Text

Gly-Gly-Val-Leu-Val-Gln-Pro-Gly (SEQ ID NO 1)

Val-Gln-Pro-Gly (SEQ ID NO 2)

Gly-Gly-Val-Leu (SEQ ID NO 3)

Leu-Val-Gln-Pro-Gly (SEQ ID NO 4)

Val-Leu-Val-Gln-Pro-Gly (SEQ ID NO 5)

Gly-Val-Leu-Val-Gln-Pro-Gly (SEQ ID NO 6)

Gly-Gly-Val-Leu-Val-Gln-Pro-Gly-Val-Gln-Pro-Gly (SEQ ID NO 7)

Gly-Gly-Val-Leu-Val-Gln-Pro-Gly-Gly-Gly-Val-Leu-Val-Gln-Pro-Gly (SEQ ID NO 8)

Organization Applicant
---------------------
Street :
City :
State :
Country :
PostalCode :
PhoneNumber :
FaxNumber :
EmailAddress :
<110> OrganizationName : SOLVAY (Société Anonyme)

Application Project
-------------------
<120> Title : PEPTIDE PRODUCTION AND PURIFICATION PROCESS
<130> AppFileReference : S 2007/39
<140> CurrentAppNumber :
<141> CurrentFilingDate : ____-__-__

Sequence
--------
<213> OrganismName : Artificial
<400> PreSequenceString :
GGVLVQPG 8
<212> Type : PRT
<211> Length : 8
SequenceName : SEQ ID NO 1
SequenceDescription :

Sequence
--------
<213> OrganismName : Artificial
<400> PreSequenceString :
VQPG 4
<212> Type : PRT
<211> Length : 4
SequenceName : SEQ ID NO 2
SequenceDescription :

Sequence
--------
<213> OrganismName : Artificial
<400> PreSequenceString :
GGVL 4
<212> Type : PRT
<211> Length : 4
SequenceName : SEQ ID NO 3
SequenceDescription :

Sequence
--------
<213> OrganismName : Artificial
<400> PreSequenceString :
LVQPG 5
<212> Type : PRT
<211> Length : 5
SequenceName : SEQ ID NO 4
SequenceDescription :

Sequence
--------
<213> OrganismName : Artificial
<400> PreSequenceString :
VLVQPG 6
<212> Type : PRT
<211> Length : 6
SequenceName : SEQ ID NO 5
SequenceDescription :

Sequence
--------
<213> OrganismName : Artificial
<400> PreSequenceString :
GVLVQPG 7
<212> Type : PRT
<211> Length : 7
SequenceName : SEQ ID NO 6
SequenceDescription :

Sequence
--------
<213> OrganismName : Artificial
<400> PreSequenceString :
GGVLVQPGVQ PG 12
<212> Type : PRT
<211> Length : 12
SequenceName : SEQ ID NO 7
SequenceDescription :

Sequence
--------
<213> OrganismName : Artificial
<400> PreSequenceString :
GGVLVQPGGG VLVQPG 16
<212> Type : PRT
<211> Length : 16
SequenceName : SEQ ID NO 8
SequenceDescription :

## Claims

1. Process for the synthesis of a Gly-Gly-Val-Leu-Val-Gln-Pro-Gly octapeptide (SEQ ID NO 1) comprising at least one peptide coupling step carried out in solution.

2. Process according to claim 1, which comprises coupling a peptide of formula :
Val-Gln-Pro-Gly-Y (SEQ ID NO 2) ;
wherein the C-terminal amino acid is protected by a carboxylic acid protecting group Y, preferably selected from alkyl esters, aryl esters, aralkyl esters and silyl groups ;
with a Leucine or a C-terminal Leucine peptide, preferably selected from formulae:
X-Gly-Gly-Val-Leu (SEQ ID NO 3), X-Gly-Val-Leu and X-Val-Leu ;
wherein the N-terminal amino acid is protected by an amino protecting group X preferably selected from allyloxycarbonyl groups, tert-butyloxycarbonyl (BOC), benzyloxycarbonyl (Z), 9-fluorenylmethyloxycarbonyl (Fmoc), 4-nitrobenzenesulfonyl (Nosyl), 2-nitrobenzenesulfenyl (Nps) and substituted derivatives;
and wherein said Leucine or C-terminal Leucine peptide is optionally activated by a carboxylic acid activating agent.

3. Process according to claim 2, wherein the C-terminal Leucine peptide is X-Gly-Gly-Val-Leu (SEQ ID NO 3).

4. Process according to claim 2 or 3 which is carried out in the presence of a carboxylic acid activating agent preferably chosen from carbodiimides, acyl halides, phosphonium salts and uronium or guanidinium salts and more preferably from isobutyl chloroformate and pivaloylchloride.

5. Process according to any of the preceding claims, wherein the peptide coupling step is carried out in a polar organic solvent preferably selected from N, N-dimethylacetamide (DMA), N, N-dimethylformamide (DMF), N-methylpyrrolidone (NMP), dimethylsulfoxide (DMSO), ethyl acetate (AcOEt), dichloromethane (DCM), methylene chloride, pyridine, chloroform, acetonitrile, dimethoxyethane, dioxane, tetrahydrofuran (THF) or mixtures thereof.

6. Process according to any of the preceding claims, wherein the coupling reaction is carried out in the presence of a base chosen from N-methylmorpholine (NMM), pyridine, triethylamine (TEA), diisopropylethylamine (DIPEA) or mixtures thereof.

7. Process according to any of the preceding claims, wherein the coupling reaction is carried out at a temperature of from -45°C to 45°C.

8. Process according to any of the preceding claims wherein after the coupling step, the solution is treated with an aqueous phase so as to provide a solution of coupled product in the aqueous phase and then, the coupled product is extracted from the aqueous phase into an organic solvent.

9. Process according to claim 8, wherein the pH value of the aqueous phase is from 1 to 5.

10. Process according to any of the preceding claims, further comprising manufacturing X-Val-Gln-Pro-Gly peptide (SEQ ID NO 2) wherein X is an amino protecting group by coupling of X-Val with Gln-Pro-Gly peptide.

11. Process according to claim 10, which comprises activating the carboxylic acid function of X-Val, preferably with N-hydroxysuccinimide.

12. Process according to claim 10 or 11, further comprising manufacturing X-Gln-Pro-Gly peptide wherein X is an amino protecting group by ring opening of X-Glp-Pro-Gly in the presence of a base.

13. Process according to any of the preceding claims, further comprising manufacturing Gly-Gly-Val-Leu peptide (SEQ ID NO 3) by coupling of Val-Leu dipeptide with X-Gly-Gly or X-Gly.

14. Process according to any of the preceding claims wherein all coupling steps are carried out in solution.

15. Process according to any of the preceding claims wherein the coupling step is carried out under persilylation conditions.

16. Process according to any of the preceding claims, according to the scheme:

Gly Gly Val Leu Val Gln Pro Gly
1 2 3 4 5 6 7 8
Z - Glp-OH
Z OSu
Z OH
Z OH
Z Gln OH
Z OSu H OH Z OSu H OH
Boc OH H OH Z OH
Boc OH H OH
Boc OH
H OH

17. Tripeptide of the formula Glp-Pro-Gly or protected peptide of the formula X-Glp-Pro-Gly, wherein X is an amino protecting group.

18. Tetrapeptide of the formula Val-Gln-Pro-Gly (SEQ ID NO 2) or protected peptide of the formula X-Val-Gln-Pro-Gly (SEQ ID NO 2), wherein X is an amino protecting group.

19. Protected tetrapeptide according to claim 18 wherein the amino protecting group X is benzyloxycarbonyl.

20. Pentapeptide of the formula Leu-Val-Gln-Pro-Gly (SEQ ID NO 4).

21. Hexapeptide of the formula Val-Leu-Val-Gln-Pro-Gly (SEQ ID NO 5).

22. Heptapeptide of the formula Gly-Val-Leu-Val-Gln-Pro-Gly (SEQ ID NO 6).

23. Dodecapeptide of the formula Gly-Gly-Val-Leu-Val-Gln-Pro-Gly-Val-Gln-Pro-Gly (SEQ ID NO 7).

24. Hexakaidecapeptide of the formula Gly-Gly-Val-Leu-Val-Gln-Pro-Gly-Gly-Gly-Val-Leu-Val-Gln-Pro-Gly (SEQ ID NO 8).

25. Modified peptide of the formula CH₃ C(=O)-NH-Gly-Gly-Val-Leu-Val-Gln-Pro-Gly (SEQ ID NO 1).

26. Use of the peptide according to anyone of claims 17 to 22 as an intermediate in peptide synthesis.

27. Process for the purification of Gly-Gly-Val-Leu-Val-Gln-Pro-Gly peptide (SEQ ID NO 1) or anyone of the peptides according to claim 17 to 25, as such or under a protected form wherein the peptide is dissolved in a first solvent and then precipitated or crystallized by the addition of a second solvent wherein the peptide is less soluble than in the first solvent.

28. Process according to claim 27, wherein the first solvent is an aqueous medium and the second solvent comprises at least one polar organic solvent preferably chosen from isopropyl ether, acetonitrile, diethyl ether, methylterbutylether, ethyl acetate, isopropyl acetate, acetone, tetrahydrofuran, dichloromethane, dioxane or mixtures thereof.

29. Process for purifying Gly-Gly-Val-Leu-Val-Gln-Pro-Gly peptide (SEQ ID NO 1) or anyone of the peptides according to claims 17 to 25 comprising subjecting a crude peptide to a chromatography operation.

30. Process according to claim 29 wherein the chromatography is a medium pressure liquid chromatography (MPLC).

31. Process according to claim 29 or 30, wherein the eluent comprises H₂O and/or CH₃CN.

32. Solution of Gly-Gly-Val-Leu-Val-Gln-Pro-Gly peptide (SEQ ID NO 1) or anyone of peptides according to claim 17 to 25 in a solvent mixture comprising water and a polar organic solvent.

33. Solution according to claim 32, wherein the polar organic solvent comprises acetonitrile.

34. Use of the solution according to claims 32 and 33 in a purification operation.

35. Process for the isolation of a Gly-Gly-Val-Leu-Val-Gln-Pro-Gly octapeptide (SEQ ID NO 1) from an aqueous medium wherein the peptide is lyophilized.
